# EUROPÄISCHE PATENTSCHRIFT

(11) **EP 1 051 388 B1**
(45) Veröffentlichungstag und Bekanntmachung des Hinweises auf die Patenterteilung: **09.04.2003**
(21) Anmeldenummer: 99906138.5
(22) Anmeldetag: 16.01.1999
(51) Int. Cl.: C07C 213/00, C07D 207/08, C07D 207/26

(54) **VERFAHREN ZUR HERSTELLUNG VON OPTISCH AKTIVEN AMINOALKOHOLEN**
METHOD FOR PRODUCING OPTICALLY ACTIVE AMINO ALCOHOLS
PROCEDE DE PREPARATION D'AMINO-ALCOOLS A ACTIVITE OPTIQUE

(30) Priorität: 31.01.1998 DE 19803892
(43) Veröffentlichungstag der Anmeldung: 15.11.2000
(73) Patentinhaber: BAYER AG, 51368 Leverkusen (DE)
(72) Erfinder: ANTONS, Stefan, D-51373 Leverkusen (DE); SCHULZE TILLING, Andreas, D-51373 Leverkusen (DE); WOLTERS, Erich, D-50939 Köln (DE)
(86) Internationale Anmeldenummer: EP9900233
(87) Internationale Veröffentlichungsnummer: WO99038838

(56) Entgegenhaltungen:
- EP-A- 0 696 575

## Beschreibung

Die vorliegende Erfindung betrifft ein verbessertes Verfahren zur Herstellung von optisch aktiven Aminoalkoholen durch Reduktion entsprechender Aminosäuren mit Rutheniumkatalysatoren.

Ein Verfahren zur Herstellung von optisch aktiven Aminoalkoholen durch katalytische Hydrierung der entsprechenden Aminosäuren an Rutheniumkatalysatoren wird in der EP-A 696 575 beschrieben. Die Enantiomerenüberschüsse der auf diese Weise hergestellten Aminoalkohole genügen noch nicht den hohen Ansprüchen für Vorprodukte für Pharmazeutika und Pflanzenschutzmittel. Zudem sind bei dem Verfahren der EP-A 696 575 die erzielbaren Ausbeuten und die benötigten Reaktionszeiten nicht besonders günstig.

Es besteht daher Bedarf nach einem gut, einfach und kostengünstig durchzuführenden Verfahren zur Herstellung von optisch aktiven Aminoalkoholen mit einem Enantiomerenüberschuß von über 99 %.

Es wurde nun ein Verfahren zur Herstellung von optisch aktiven Aminoalkpholen aus optisch aktiven Aminosäuren durch Reduktion mit Wasserstoff in Gegenwart von Ruthenium enthaltenden Katalysatoren gefunden, das dadurch gekennzeichnet ist, daß man unter Zusatz von Säuren arbeitet.

In das erfindungsgemäße Verfahren kann man z.B. optisch aktive Aminosäuren der Formel (I) einsetzen 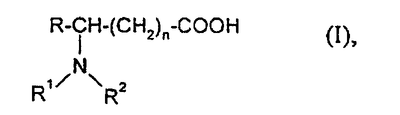 in der
R für geradkettiges oder verzweigtes C₁-C₁₂-Alkyl, C₇-C₁₂-Aralkyl oder C₆-C₁₀-Aryl steht, die gegebenenfalls durch NR³R⁴, OH oder COOH substituiert sein können,
R¹, R², R³ und R⁴ unabhängig voneinander jeweils für Wasserstoff, geradkettiges oder verzweigtes C₁-C₁₂-Alkyl oder C₃-C₈-Cycloalkyl stehen, wobei
   R¹ und R² sowie R³ und R⁴ unabhängig voneinander jeweils gemeinsam auch für -(CH₂)ₘ- mit m = einer ganzen Zahl von 4 bis 7 stehen können und
   wobei R und R¹ gemeinsam auch für -(CH₂)ₒ- mit o = einer ganzen Zahl von 2 bis 6 stehen können und
n für Null oder eine ganze Zahl von 1 bis 5 steht,
   und optisch aktive Aminoalkohole der Formel (II) erhalten 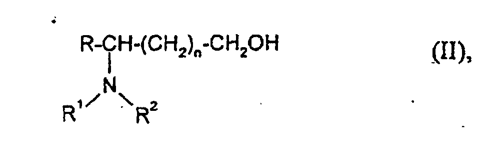
in der

R, R¹, R² und n die bei Formel (I) angegebene Bedeutung haben.

In den Formeln (I) und (II) steht R vorzugsweise für geradkettiges oder verzweigtes C₁-C₄-Alkyl, das gegebenenfalls durch NR³R⁴, OH oder COOH substituiert ist oder für Benzyl. R¹, R², R³ und R⁴ stehen vorzugsweise unabhängig voneinander jeweils für Wasserstoff oder geradkettiges oder verzweigtes C₁-C₄-Alkyl, wobei R¹ und R² sowie R³ und R⁴ unabhängig voneinander jeweils gemeinsam auch für -(CH₂)ₘ- mit m = 4 oder 5 stehen können und wobei R und R¹ gemeinsam auch für -(CH₂)ₒ- mit o = 3 oder 4 stehen können. n steht vorzugsweise für Null, 1 oder 2.

Außerdem ist es bevorzugt, daß R¹ und R² gleich sind oder gemeinsam für eine Polymethylenbrücke stehen. Gleiches gilt für R³ und R⁴.

Besonders bevorzugte Aminosäuren der Formel (I) sind Alanin, valin, Leucin, Isoleucin, Threonin, Ornithin, Asparaginsäure, Glutaminsäure, Phenylalanin und Prolin.

Das erfindungsgemäße Verfahren kann z.B. unter Zusatz von 0,5 bis 1,5 Äquivalenten einer organischen oder anorganischen Säure bezogen auf 1 Äquivalent basische Gruppen in der eingesetzten optisch aktiven Aminosäure durchgeführt werden. Als Säuren sind anorganische Säuren bevorzugt, insbesondere Schwefelsäure, Salzsäure und Phosphorsäure. Die Säuren können z.B. als solche, in Form wäßriger Lösungen oder in Form ihrer separat hergestellten Addukte mit den eingesetzten Aminosäuren, z.B. als Sulfate, Hydrogensulfate, Hydrochloride, Phosphate, Dihydrogenphosphate oder Monohydrogenphosphate eingesetzt werden.

Die Menge der zuzusetzenden Säure wird vorzugsweise so gewählt, daß nach deren Zusatz alle Carboxylgruppen der eingesetzten Aminosäure in protonierter Form vorliegen. Vorzugsweise setzt man pro Äquivalent basische Gruppen der Aminosäuren 1 bis 1,3 Äquivalente organische oder anorganische Säure ein.

Als Katalysatoren für das erfindungsgemäße Verfahren kommen z.B. Ruthenium, bimetallische Ruthenium/Metall X-Katalysatoren und trimetallische Ruthenium/-Metall X/Metall Y-Katalysatoren in Frage, die alle als solche oder aufgebracht auf einem Trägermaterial zum Einsatz gelangen können. Bei den Metallen X und Y kann es sich beispielsweise je um eines aus der Gruppe der Metalle und Übergangsmetalle mit Ordnungszahlen im Bereich 23 bis 82 handeln. Die Katalysatoren können Ruthenium und gegebenenfalls die Metalle X und gegebenenfalls die Metalle Y in verschiedener Form enthalten, beispielsweise in elementarer Form, in Form von Verbindungen des Rutheniums oder des Rutheniums und der Metalle X oder des Rutheniums und der Metalle X und Y oder in Form einer intermetallischen Verbindung aus Ruthenium und dem Metall X und gegebenenfalls dem Metall Y. Wenn die Katalysatoren nicht aufgebracht auf einem Trägermaterial zum Einsatz gelangen, können sie beispielsweise in kolloidaler Form oder als feinteiliger Feststoff vorliegen. Beispiele für Katalysatoren sind fein verteilte Ruthenium/Rhenium-, Ruthenium/Osmium-, Ruthenium/Eisen-, Ruthenium/Cobalt-, Ruthenium/Rhodium-, Ruthenium/Palladium-, Ruthenium/Platin-, Ruthenium/Kupfer-, Ruthenium/Zink-, Ruthenium/Silber-, Ruthenium/Zinn-, Ruthenium/Germanium-, Ruthenium/-Gallium-, Ruthenium/Blei-, Ruthenium/Rhenium/Kupfer-, Ruthenium/Rhenium/-Silber- und Ruthenium/Rhenium/Zinn-Partikel z.B. in metallischer Form oder in Form ihrer Oxide, Hydroxide, Halogenide, Nitrate, Carboxylate, Acetylacetonate oder als Aminkomplexe.

Als Trägermaterial kommen beispielsweise Kohlen, Ruße, Graphite, Aluminiumoxide, Siliciumdioxide, Silikate, Zeolithe und Tonerden in Frage. Trägerkatalysatoren können beispielsweise 1 bis 50 Gew.-% Metall in elementarer Form oder in Form von Verbindungen enthalten.

Die einzusetzenden Katalysatoren können gegebenenfalls durch eine Behandlung mit Schwefelverbindungen, z.B. mit Thiocther, modifiziert worden sein.

Bevorzugt sind Katalysatoren, die Ruthenium und Rhenium ohne Träger enthalten und als bimetallische Katalysatorpartikel eine hohe spezifische Oberfläche aufweisen, z.B. eine solche von 50 bis 150 m²/g. Derartige Katalysatoren kann man z.B. herstellen, indem man auf einem Rutheniumoxidhydrat mit hoher Oberfläche (z.B. 50 bis 300 m²/g) Rhenium aus einer Rheniumlösung durch Einwirkung von Wasserstoff reduktiv abscheidet. Dabei wird ein bimetallischer Katalysator mit hoher Oberfläche und innigem Kontakt der beiden Metalle erhalten. Überraschenderweise kann die Reduktion des gelösten Rheniums in Gegenwart von Ruthenium bei wesentlich niedrigeren Temperaturen erfolgen als bei Abwesenheit von Ruthenium. Grundsätzlich kann die Abscheidung eines zweiten Metalls bei der Katalysatorherstellung oder in situ bei der Hydrierreaktion erfolgen.

Bezogen auf 1 Mol eingesetzte optisch aktive Aminosäure kann man als Katalysator z.B. 0,1 bis 10g Metall oder Metallverbindungen oder 1 bis 50 g Trägerkatalysatoren, die Metall oder Metallverbindungen enthalten, einsetzen.

Das erfindungsgemäße Verfahren wird im allgemeinen in Gegenwart eines Lösungsmittels für die optisch aktiven Aminosäuren und optisch aktiven Aminoalkohole durchgeführt. Als Lösungsmittel kommen beispielsweise Wasser, mit Wasser mischbare organische Lösungsmittel und Gemische aus beiden in Frage. Als mit Wasser mischbare Lösungsmittel seien niedere Alkohole und mit Wasser mischbare Ether genannt. Bevorzugte Lösungsmittel sind Wasser und Gemische, die Wasser und niedrige Alkohole oder Tetrahydrofuran enthalten.

Das erfindungsgemäße Verfahren kann man z.B. bei Temperaturen im Bereich 0 bis 150°C und Drucken im Bereich 5 bis 300 bar durchführen. Bevorzugt sind Temperaturen von 0 bis 130°C und Drücke von 10 bis 280 bar. Besonders bevorzugt werden Temperaturen von 30 bis 80°C und Drücke von 150 bis 250 bar. Man kann gegebenenfalls auch so verfahren, daß man die'Reduktion mit Wasserstoff bei einem relativ niedrigen Druck beginnt, z.B. bei 50 bis 150 bar, und sie dann bei relativ höheren Drucken zu Ende führt, z.B. 150 bis 300 bar. Die Reaktion ist beendet, wenn kein Wasserstoff mehr aufgenommen wird, was im allgemeinen nach 1 bis 10 Stunden der Fall ist. Bei niedrigen Drucken und niedrigen Temperaturen kann die Reaktionszeit auch länger sein.

Zur Aufarbeitung des Reaktionsgemisches kann man beispielsweise zunächst abkühlen, den Katalysator z.B. durch Filtration abtrennen, die vorhandenen leicht flüchtigen Bestandteile (i.a. Lösungsmittel und Reaktionswasser) durch Destillation, gegebenenfalls unter vermindertem Druck, teilweise oder ganz entfernen, aus dem Rückstand mit Base (z.B. wäßriger Alkalilauge oder alkoholischer Alkoholatlösung) den Aminoalkohol aus seinem Salz freisetzen, das ausgefallene Salz abtrennen und das Filtrat im Vakuum fraktionieren. Den abgetrennten Katalysator kann man wiederverwenden, ebenso das Lösungsmittel.

Das erfindungsgemäße Verfahren kann kontinuierlich, halbkontinuierlich oder diskontinuierlich durchgeführt werden. Als Reaktoren eignen sich z.B. Rührkessel und Rieselphasereaktoren. Vorteilhaft wird das Verfahren als Batch-Zulaufverfahren durchgeführt, wobei der Katalysator im Lösungsmittel vorgelegt wird und die saure Lösung der Aminosäure in der Menge zugepumpt wird, wie sie bei der Hydrierung verbraucht wird. Hierdurch läßt sich die Konzentration des Aminosäuresalzes im Reaktor auf einem niedrigen Niveau halten, was sich auf die Katalysatorstandzeit und die Ausbeute positiv auswirkt und die Korrosivität des Reaktionsmediums erniedrigt

Das erfindungsgemäße Verfahren hat die Vorteile, daß bei der Hydrierung von optisch aktiven Aminosäuren mit einem Ruthenium-haltigen Katalysator optisch aktive Aminoalkohole mit höherer Reinheit, mit höherem Enantiomerenübcrschuß, in höherer Ausbeute, bei niedrigeren Temperaturen und in kürzeren Reaktionszeiten als ohne Zusatz von Säure zugänglich sind. Weiterhin wurde gefunden, daß ein Katalysator, der zusätzlich zu Ruthenium mindestens ein weiteres Metall enthält, insbesondere Rhenium, eine wesentlich höhere Performance in der Hydrierung von optisch aktiven Aminosäuren hat als ein monometallischer Rutheniumkatalysator. Diese Vorteile des erfindungsgemäßen Verfahrens sind außergewöhnlich überraschend.

### Beispiele

Prozentangaben sind, soweit nichts anderes gesagt ist, Gewichtsprozente.

### Beispiel 1

In einem 0,71 Edelstahl-Autoklaven wurden zu einer Suspension von 33,6 g wasserfeuchtem Rutheniumoxidhydrat (8,93 % Ru) mit einer Oberfläche von 210 m²/g in 100 ml Wasser 3,9 g Rhenium(VII)oxid (76,87 % Re) hinzugefügt. Nach dem Spülen mit Stickstoff wurden 100 bar Wasserstoff aufgedrückt und auf 120°C aufgeheizt. Danach wurde der Wasserstoffdruck auf 150 bar erhöht und 1 Stunde bei 120°C gerührt.

Zu der erhaltenen Suspension von Ru-Re-Mohr wurde eine Lösung von 60,0 g L-Alanin und 32,9 g Schwefelsäure in 370 g Wasser hinzugefügt. Nach dem Spülen mit Stickstoff wurde der Autoklav verschlossen und 100 bar Wasserstoff aufgedrückt. Innerhalb von 30 Minuten wurde die Temperatur auf 60°C angehoben und der Wasserstoffdruck auf 200 bar erhöht. Nach 7 Stunden Reaktionszeit war die Wasserstoffaumahme beendet. Es wurde auf Raumtemperatur abgekühlt, entspannt, aus dem Reaktionsgemisch der Katalysator durch Filtration abgetrennt und aus dem Filtrat das Wasser abdestilliert. Der Rückstand wurde mit 59,7 g 45 %iger Natronlauge auf einen pH-Wert von 11,4 eingestellt und bei 50 mbar fraktioniert destilliert. Es wurden 35,7 g reines L-Alaninol (Kp.: 94°C/50 mbar), ee >99,9 % erhalten. Dies entspricht einer Ausbeute von 71 % der Theorie. Hier und in den anderen Beispielen wurde der ee-Wert gaschromatographisch bestimmt.

### Beispiel 2

Es wurde wie in Beispiel 1 verfahren, jedoch wurde die Hydrierung bei 80°C durchgeführt. Die Wasserstoffaufnahme war nach 5 Stunden beendet. Es wurden 35,0 g reines L-Alaninol erhalten, was einer Ausbeute von 69 % der Theorie entspricht. Der ee-Wert war >99,9 %.

### Beispiel 3

Es wurde wie in Beispiel 1 verfahren, jedoch wurde die Hydrierung bei 100°C durchgeführt. Die Wasserstoffaufnahme war nach 3 Stunden beendet. Es wurden 26,5 g reines L-Alaninol erhalten, was einer Ausbeute von 53 % der Theorie entspricht. Der ee-Wert betrug 90,3 %.

### Beispiel 4

Beispiel 1 wurde 5 mal wiederholt, wobei stets der beim vorhergehenden Ansatz abgetrennte Katalysator wieder eingesetzt wurde. Es wurde keine Änderung am Enantiomerenüberschuß des erhaltenen L-Alaninols festgestellt.

### Beispiel 5

Es wurde verfahren wie in Beispiel 1, jedoch wurde statt L-Alanin die molmäßig entsprechende Menge L-Valin eingesetzt. Nach der Destillation wurden 33,5 g reines L-Valaninol erhalten. Dies entspricht einer Ausbeute von 49 % der Theorie. Der ee-Wert lag bei über 99,9 %.

### Beispiel 6

Es wurde verfahren wie in Beispiel 1, jedoch wurde bei der Katalysatorherstellung kein Rhenium(VII)oxid zugesetzt. Die Hydrierzeit betrug 35 Stunden. Nach Aufarbeitung wurden 36,2 g reines L-Alaninol erhalten, was einer Ausbeute von 72 % der Theorie entspricht. Der ee-Wert lag bei über 99,9 %.

### Beispiel 9 (Vergleichsbeispiel)

Es wurde verfahren wie in Beispiel 2, jedoch wurde keine Schwefelsäsure zugesetzt und nach Abtrennung des Katalysators auch keine Natronlauge. Nach 20 Stunden wurde kein Wasserstoff mehr aufgenommen. Nach der Aufarbeitung wurden 7,3 g reines L-Alaninol erhalten, was einer Ausbeute von 15 % der Theorie entspricht. Der ee-Wert lag bei 94,2 %.

### Beispiel 10

In einem 0,71 -Edelstahl-Autoklaven wurden 58,8 g RuO₂ wasserfeucht (10,19 % Ru), 7,8 g Re₂O₇ (76,9 % Re) und 100 ml Wasser vorgelegt. Nach dem Spülen mit Stickstoff wurden 100 bar H₂ aufgedrückt und unter Rühren (800 UPM) auf 120°C geheizt. Nach Erreichen dieser Temperatur wurde der H₂-Druck auf 150 bar erhöht und der Katalysator bei 120°C eine Stunde vorreduziert.

Zu der erhaltenen Suspension von Ru-Re-Mohr wurde nach dem Abkühlen des Autoklaven auf 70°C eine Lösung von 138 g L-Alanin (99,2 %-ig.) (1,54 mol) in 256 g 30 %-ig. wäßrigen H₂SO₄ hinzugefügt und der H₂-Druck auf 200 bar erhöht. Nach 8 Stunden war die Wasserstoffaufnahme beendet.

Nach dem Abkühlen auf Raumtemperatur wurde der Katalysator vom Reaktionsgemisch abfiltriert und mit 23 g Wasser gewaschen.

Es wurden 548,4 g Rohlösung erhalten, von der am Rotationsverdampfer bei 70°C und 250 - 30 mbar 300 g Wasser abdestilliert wurden.

Zum Rückstand wurden unter Rühren bei Raumtemperatur innerhalb von 30 min 279 g 30 %-ig. methanolische Natriummethylat-Lösung zugetropft. Die Temperatur stieg dabei auf etwa 35°C. Die erhaltene Suspension wurde noch 15 min nachgerührt.

Das ausgefallene Natriumsulfat (116,3 g feucht) wurde abgesaugt und dreimal mit je 30 g Methanol gewaschen. Der Rückstand wurde über eine 30 cm Vigreuxkolonne destilliert.

Nach einem Vorlauf von 370 g Methanol/Wasser wurden bei 93-96°C und 50 mbar 94 g (S)-2-Aminopropanol (> 99 %-ig nach GC) erhalten.

Dies entspricht einer Ausbeute von 80 % d. Th..

Der ee-Wert betrug 99,9 %.

### Beispiele 11-20

In gleicher Weise wie in Beispiel 10 wurden die in Tabelle 1 aufgeführten Aminosäuren hydriert (Reaktionsbedingungen nicht optimiert).

**Tabelle**

| **Beispiel** | **Edukt** | **Produkt** | **Siedepunkt** | **Ausbeute** | **ee-Wert** |
|---|---|---|---|---|---|
| 11 | LValin | L-Valinol | 109°C/50 mbar | 72 % | >99,9 % |
| 12 | L-Leucin | L-Leucinol | 56°C/1 mbar | 48 % | >99,9 % |
| 13 | L-Isoleucin | L-Isoleucinol | 63°C/1 mbar | 56 % | >99,9 % |
| 14 | L-tert.-Leucin | L-tert.-Leucinol | 67°C/2 mbar | 31 % | >99,9 % |
| 15 | L-Threonin | L.Threoninol | 111°C/0,4 mbar | 61 % | 98,7 % |
| 16 | L-Asparaginsäure | L-Asparaginol | 130°C/0,5 mbar | 60 % | 98,9 % |
| 17 | L-Glutaminsäure | L-Glutaminol | 145°C/0,2 mbar | 58 % | 98,3 % |
| 18 | L-Prolin | L-Prolinol | 53°C/0,4 mbar | 45 % | 99,2 % |
| 19 | L-Pyroglutaminsäure | L-Pyroglutaminol | 168°C/0,4 mbar | 65 % | 98,3 % |
| 20 | L-Phenylalanin | L-Cyclohexylalanin | 110°C/1 mbar | 32 % | 99,3 % |

## Patentansprüche

1. Verfahren zur Herstellung von optisch aktiven Aminoalkoholen aus optisch aktiven Aminosäuren durch Reduktion mit Wasserstoff in Gegenwart von Ruthenium enthaltenden Katalysatoren, **dadurch gekennzeichnet, daß** man unter Zusatz von Säuren arbeitet.

2. Verfahren nach Anspruch 1, **dadurch gekennzeichnet, daß** man als optisch aktive Aminosäuren solche der Formel (I) einsetzt 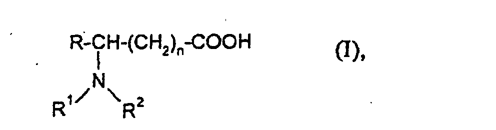 in der
R für geradkettiges oder verzweigtes C₁-C₁₂-Alkyl, C₇-C₁₂-Aralkyl oder C₆-C₁₀-Aryl steht, die gegebenenfalls durch NR³R⁴, OH oder COOH substituiert sein können,
R¹, R², R³ und R⁴ unabhängig voneinander jeweils für Wasserstoff, geradkettiges oder verzweigtes C₁-C₁₂-Alkyl oder C₃-C₈-Cycloalkyl stehen, wobei
R¹ und R² sowie R³ und R⁴ unabhängig voneinander jeweils gemeinsam auch für -(CH₂)ₘ- mit m = einer ganzen Zahl von 4 bis 7 stehen können und
wobei R und R¹ gemeinsam auch für -(CH₂)ₒ- mit o = einer ganzen Zahl von 2 bis 6 stehen können und
n für Null oder eine ganze Zahl von 1 bis 5 steht,
und optisch aktive Aminoalkohole der Formel (II) erhält 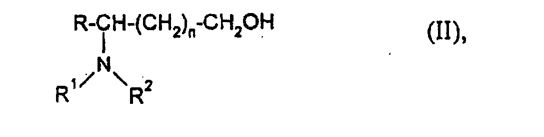 in der
R, R¹, R² und n die bei Formel (I) angegebene Bedeutung haben.

3. Verfahren nach Ansprüchen 1 und 2, **dadurch gekennzeichnet, daß** man bezogen auf 1 Äquivalent basische Gruppen in der eingesetzten optisch aktiven Aminosäure 0,5 bis 1,5 Äquivalente einer organischen oder anorganischen Säure einsetzt.

4. Verfahren nach Ansprüchen 1 bis 3, **dadurch gekennzeichnet, daß** man Schwefelsäure, Salzsäure oder Phosphorsäure einsetzt.

5. Verfahren nach Ansprüchen 1 bis 4, **dadurch gekennzeichnet, daß** man als optisch aktive Aminosäuren der Formel (I) Alanin, Valin, Leucin, Isoleucin, Threonin, Ornithin, Asparaginsäure, Phenylalanin oder Prolin einsetzt.

6. Verfahren nach Ansprüchen 1 bis. 5, **dadurch gekennzeichnet, daß** man als Katalysatoren Ruthenium, bimetallische Ruthenium/Metall X-Katalysatoren oder trimetallische Ruthenium/Metall X/Metall Y-Katalysatoren als solche oder aufgebracht auf einen Träger einsetzt, wobei es sich bei den Metallen X und Y um je eines aus der Gruppe der Metalle und Übergahgsmetalle mit Ordnungszahlen im Bereich 23 bis 82 handelt.

7. Verfahren nach Ansprüchen 1 bis 6, **dadurch gekennzeichnet, daß** man den Katalysator in metallischer Form oder in Form von Oxiden, Hydroxiden, Halogeniden, Nitraten, Carboxylaten, Acetylacetonaten oder Aminkomplexen einsetzt.

8. Verfahren nach Ansprüchen 1 bis 7, **dadurch gekennzeichnet, daß** der eingesetzte Katalysator Ruthenium und Rhenium ohne Träger enthält.

9. Verfahren nach Ansprüchen 1 bis 8, **dadurch gekennzeichnet, daß** man es in Gegenwart von Wasser, von einem mit Wasser mischbaren organischen Lösungsmittel oder von einem Gemisch aus beiden durchführt.

10. Verfahren nach Ansprüchen 1 bis 9, **dadurch gekennzeichnet, daß** man es bei Temperaturen im Bereich von 0 bis 150°C und Drucken im Bereich von 5 bis 300 bar durchführt.

## Claims

1. Process for preparing optically active amino alcohols from optically active amino acids by reduction with hydrogen in the presence of ruthenium-containing catalysts, **characterized in that** the reduction is carried out with addition of acids.

2. Process according to Claim 1, **characterized in that** the optically active amino acids used have the formula (I) 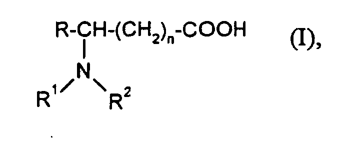 where
R represents straight-chain or branched C₁-C₁₂-alkyl, C₇-C₁₂-aralkyl or C₆-C₁₀-aryl, which may each be substituted by NR³R⁴, OH or COOH,
R¹, R², R³ and R⁴ each represent, independently of one another, hydrogen, straight-chain or branched C₁-C₁₂-alkyl or C₃-C₈-cycloalkyl, where
R¹ and R² and also R³ and R⁴ may in each case, independently of one another, together also represent -(CH₂)ₘ-, where m = an integer from 4 to 7, and
where R and R¹ may together also represent -(CH₂)ₒ-, where o = an integer from 2 to 6, and
n represents zero or an integer from 1 to 5,
and the optically active amino alcohols obtained have the formula (II) 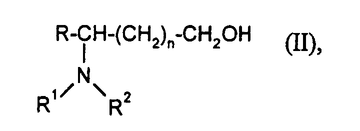 where
R, R¹, R² and n are as defined for formula (I).

3. Process according to Claims 1 and 2, **characterized in that** from 0.5 to 1.5 equivalents of an organic or inorganic acid are used per 1 equivalent of basic groups in the optically active amino acid used.

4. Process according to Claims 1 to 3, **characterized in that** sulphuric acid, hydrochloric acid or phosphoric acid is used.

5. Process according to Claims 1 to 4, **characterized in that** the optically active amino acid of the formula (I) used is alanine, valine, leucine, isoleucine, threonine, ornithine, aspartic acid, phenylalanine or proline.

6. Process according to Claims 1 to 5, **characterized in that** the catalysts used are ruthenium, bimetallic ruthenium/metal X catalysts or trimetallic ruthenium/metal X/metal Y catalysts as such or applied to a support, where the metals X and Y are each a metal selected from the group of metals and transition metals having atomic numbers in the range from 23 to 82.

7. Process according to Claims 1 to 6, **characterized in that** the catalyst is used in metallic form or in the form of oxides, hydroxides, halides, nitrates, carboxylates, acetylacetonates or amine complexes.

8. Process according to Claims 1 to 7, **characterized in that** the catalyst used contains ruthenium and rhenium without a support.

9. Process according to Claims 1 to 8, **characterized in that** it is carried out in the presence of water, of a water-miscible organic solvent or of a mixture of the two.

10. Process according to Claims 1 to 9, **characterized in that** it is carried out at temperatures in the range from 0 to 150°C and pressures in the range from 5 to 300 bar.

## Revendications

1. Procédé pour la préparation d'aminoalcools à l'état d'isomères optiques à partir d'aminoacides à l'état d'isomères optiques par réduction à l'aide de l'hydrogène en présence de catalyseurs contenant du ruthénium, **caractérisé en ce que** l'on opère avec adjonction d'acides.

2. Procédé selon la revendication 1, **caractérisé en ce que** les aminoacides isomères optiques mis en oeuvre répondent à la formule (I) 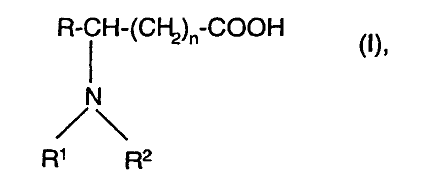 dans laquelle
R représente un groupe alkyle à chaîne droite ou ramifiée en C₁-C₁₂, aralkyle en C₇-C₁₂ ou aryle en C₆-C₁₀, ces groupes pouvant le cas échéant porter des substituants NR³R⁴, OH ou COOH,
R¹, R², R³ et R⁴ représentent chacun, indépendamment les uns des autres, l'hydrogène, un groupe alkyle à chaîne droite ou ramifiée en C₁-C₁₂ ou cycloalkyle en C₃-C₈,
R¹ et R² d'une part, R³ et R⁴ d'autre part, pouvant former ensemble, indépendamment, un groupe -(CH₂)ₘ- pour lequel m est un nombre entier allant de 4 à 7 et
R et R¹ pouvant également former ensemble un groupe -(CH₂)ₒ-pour lequel o est un nombre entier allant de 2 à 6 et
n est égal à 0 ou représente un nombre entier allant de 1 à 5,
et on obtient des aminoalcools isomères optiques de formule (II), 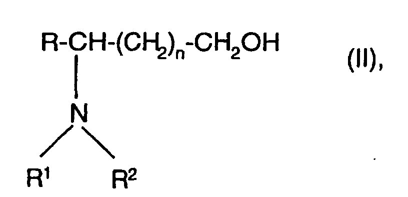 dans laquelle
R, R¹, R² et n ont les significations indiquées en référence à la formule (I).

3. Procédé selon les revendications 1 et 2, **caractérisé en ce que** l'on utilise de 0,5 à 1,5 équivalent d'un acide organique ou minéral pour un équivalent de groupes basiques de l'aminoacide isomère optique mis en oeuvre.

4. Procédé selon les revendications 1 à 3, **caractérisé en ce que** l'on utilise l'acide sulfurique, l'acide chlorhydrique ou l'acide phosphorique.

5. Procédé selon les revendications 1 à 4, **caractérisé en ce que** les aminoacides isomères optiques de formule (I) mis en oeuvre sont l'alanine, la valine, la leucine, l'isoleucine, la thréonine, l'ornithine, l'acide aspartique, la phénylalanine ou la proline.

6. Procédé selon les revendications 1 à 5, **caractérisé en ce que** l'on utilise en tant que catalyseurs le ruthénium, des catalyseurs bimétalliques ruthénium/métal X ou des catalyseurs trimétalliques ruthénium/métal X/métal Y, tels quels ou appliqués sur un support, les métaux X et Y appartenant tous deux au groupe des métaux et métaux de transition de numéro atomique 23 à 82.

7. Procédé selon les revendications 1 à 6, **caractérisé en ce que** le catalyseur est mis en oeuvre à l'état métallique ou à l'état d'oxydes, d'hydroxydes, d'halogénures, de nitrates, de carboxylates, d'acétylacétonates ou de complexes d'aminés.

8. Procédé selon les revendications 1 à 7, **caractérisé en ce que** le catalyseur mis en oeuvre consiste en ruthénium et rhénium sans support.

9. Procédé selon les revendications 1 à 8, **caractérisé en ce que** l'on opère en présence d'eau, d'un solvant organique miscible à l'eau ou d'un mélange d'eau et d'un solvant organique miscible à l'eau.

10. Procédé selon les revendications 1 à 9, **caractérisé en ce que** l'on opère à des températures dans l'intervalle de 0 à 150°C sous des pressions dans l'intervalle de 5 à 300 bar.
